# EUROPEAN PATENT APPLICATION

(11) **EP 2 815 782 A1**
(43) Date of publication of application: **24.12.2014**
(21) Application number: 13748561.1
(22) Date of filing: 14.02.2013
(51) Int. Cl.: A61M 25/06, A61M 25/02

(54) **INDWELLING NEEDLE DEVICE**

(30) Priority: 17.02.2012 JP 2012032843
(71) Applicant: JMS Co., Ltd., Hiroshima-shi, Hiroshima 730-8652 (JP)
(72) Inventor: ITO, Toru, Hiroshima-shi, Hiroshima 730-8652 (JP); SETOGUCHI, Daisuke, Hiroshima-shi, Hiroshima 730-8652 (JP)
(74) Representative: Piésold, Alexander James
(86) International application number: PCT/JP2013/053542
(87) International publication number: WO 2013/122149

(57) **Abstract**

A hub (40) housed within an inner cavity (24) of a shield (20) can be displaced from an initial position at which an inner needle (50) penetrates an outer needle (30) and protrudes from a leading end of the outer needle to a retracted position at which the inner needle is housed within the inner cavity of the shield. When a stopper (70) is inserted into the inner cavity of the shield and a leading end thereof is caused to abut against the hub located at the initial position, a rear end portion (71) of the stopper is exposed behind the shield. In a state in which the leading end of the stopper abuts against the hub located at the initial position, the stopper can be pulled out of the shield with one hand, by grasping the rear end portion while pressing a finger against the rear end, or the vicinity thereof, of the shield.

## Description

### Technical Field

The present invention relates to an indwelling needle device that includes a soft outer needle and a hard inner needle, and that is configured such that it can be inserted into a patient in a state in which a leading end of the inner needle protrudes from a leading end of the outer needle and then the inner needle can be retracted from the outer needle.

### Background Art

Indwelling needle devices are used widely for treatments such as infusion, blood transfusion, and extracorporeal blood circulation. In such treatments, leaving a metal needle inside a blood vessel may injure the blood vessel. Thus, indwelling needle devices are known that include a soft outer needle and a hard inner needle. The outer needle and the inner needle are inserted into a blood vessel of a patient in a state in which a leading end of the inner needle protrudes from a leading end of the outer needle, and then the inner needle is retracted from the outer needle, so that only the outer needle is left inside the patient. The possibility that the left soft outer needle will injure the blood vessel of the patient is low.

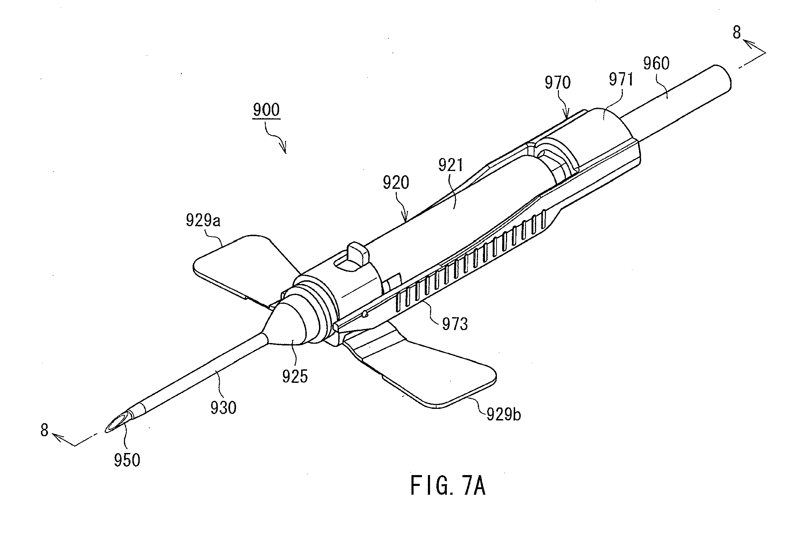

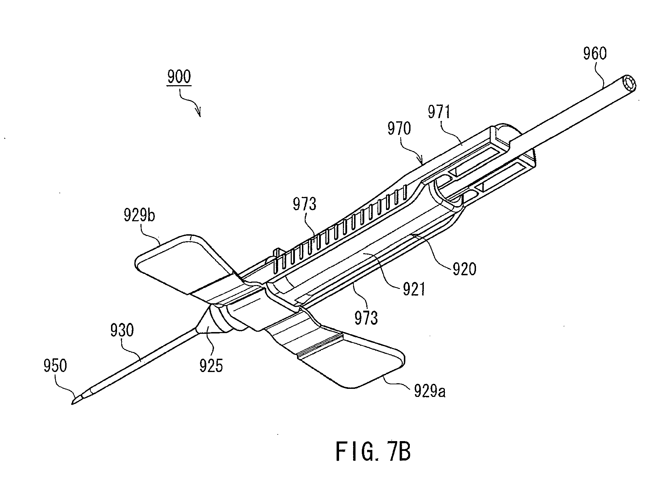

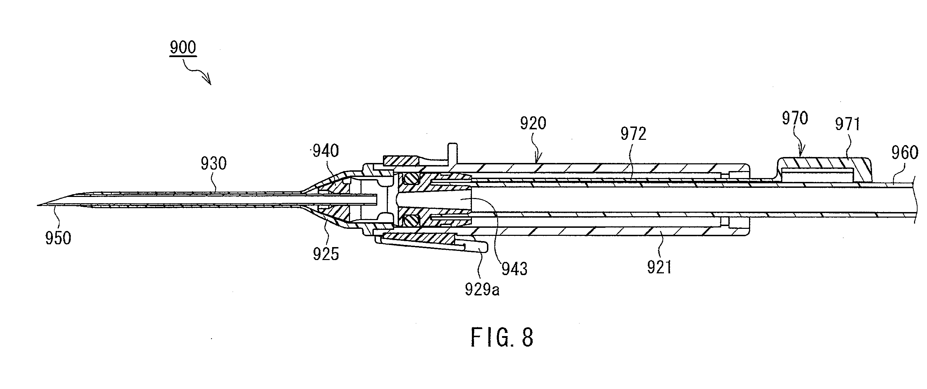

FIG. 7A is a perspective view of an example of such a conventional indwelling needle device 900 (see Patent Document 1, for example) as seen from above. FIG. 7B is a perspective view thereof as seen from below. FIG. 8 is a cross-sectional view of the conventional indwelling needle device 900 taken along a vertical plane containing line 8-8 in FIG. 7A and seen in the direction of arrows 8. For the sake of convenience of description, a side that is inserted into the patient (the left side in FIGS. 7A, 7B, and 8) is referred to as a "front side", and a side that is opposite this side is referred to as a "rear side".

The indwelling needle device 900 includes a shield 920 configured by a shield tube 921 that has an approximately cylindrical shape, and an outer hub 925 that is fixed to an end (front end) of the shield tube 921. A soft outer needle 930 is fixed to a front end of the outer hub 925.

A pair of wings 929a and 929b are provided on an outer circumferential face of the shield tube 921 in the vicinity of its outer hub 925 side end. The wings 929a and 929b are flexible, and can be swung up and down.

A hub 940 is inserted in an inner cavity of the shield 920 so as to be movable in a longitudinal direction (i.e., front-rear direction) of the shield 920. A hard inner needle 950 made of metal is fixed to a front end of the hub 940, and one end of a flexible tube 960 is connected to a rear end of the hub 940. The inner needle 950 and the tube 960 are in communication with each other via a longitudinal penetration path 943 that penetrates the hub 940 in the front-rear direction.

In FIGS. 7A, 7B, and 8, the hub 940 is located on the front end side of the inner cavity of the shield 920. This position of the hub 940 relative to the shield 920 is referred to as an "initial position". At the initial position, the inner needle 950 held by the hub 940 penetrates the outer needle 930, and the leading end of the inner needle 950 protrudes to the outside from the leading end of the outer needle 930.

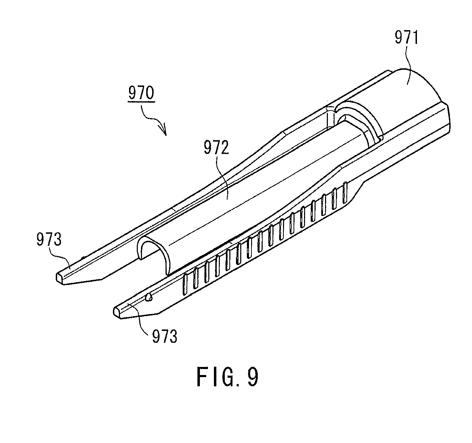

In order to maintain the hub 940 at the initial position, a stopper 970 is used. FIG. 9 is a perspective view of the stopper 970. An approximately semi-cylindrical insertion portion 972 and a pair of fixing portions 973 extend from an approximately semi-cylindrical base portion 971. The insertion portion 972 is disposed between the pair of fixing portions 973, and these portions are parallel to one another.

As shown in FIG. 8, the insertion portion 972 of the stopper 970 is inserted from the rear end of the shield tube 921. When a leading end of the insertion portion 972 hits the rear end of the hub 940 and pushes the hub 940 toward the front side, the hub 940 can be disposed at the initial position.

The indwelling needle device 900 is used as follows.

First, the inner needle 950 and the outer needle 930 are inserted into a blood vessel of the patient in a state in which the hub 940 is kept at the initial position (insertion operation).

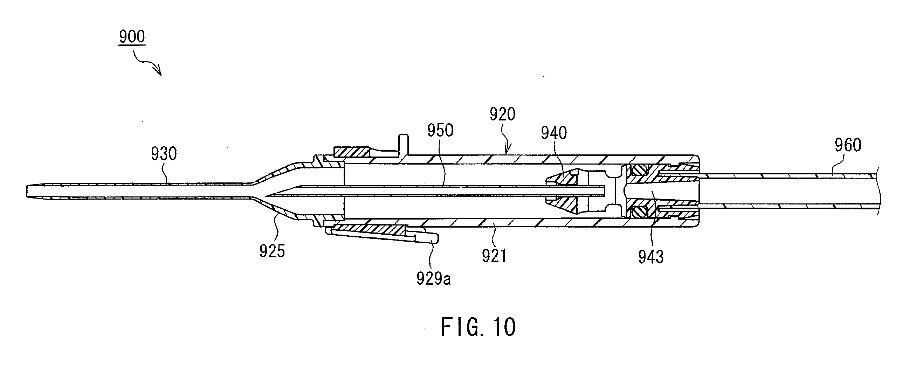

Subsequently, the stopper 970 is pulled out of the shield 920, and then the tube 960 is pulled from the shield 920 (retraction operation). The stopper 970 may be pulled out of the shield 920 at the same time that the tube 960 is pulled. Accordingly, the hub 940 and the inner needle 950 are moved together with the tube 960 toward the rear side relative to the shield 920, and the inner needle 950 is housed within the shield 920 as shown in FIG. 10. The position of the hub 940 relative to the shield 920 shown in FIG. 10 is referred to as a "retracted position". In this state, the indwelling needle device 910 is fixed to the patient using an adhesive tape or the like. Only the soft outer needle 930 is left inside the patient in a state in which it is inserted in the patient.

### Citation List

### Patent Document

Patent Document 1: Japanese Patent No. 4506834

### Disclosure of Invention

### Problem to be Solved by the Invention

According to the conventional indwelling needle device 900, the insertion operation that inserts the inner needle 950 and the outer needle 930 into the patient can be performed while holding the indwelling needle device 900 with one hand. However, the following retraction operation that moves the hub 940 from the initial position to the retracted position has to be performed by, while holding the shield 920 with one of the hands so as to maintain the state in which the outer needle 930 is inserted in the patient, pulling the tube 960 with the other hand. In this manner, the retraction operation has to be performed with both hands, although the insertion operation can be performed with one hand.

However, in medical practice, there are cases in which an operator operating the indwelling needle device 900 has to press or hold an arm or the like of a patient with one hand. Accordingly, it is desirable that not only the insertion operation but also the retraction operation can be performed with one hand.

It is an object of the present invention to provide an indwelling needle device in which not only an insertion operation that inserts an inner needle and an outer needle into a patient but also its following retraction operation that houses the inner needle within a shield can be performed with one hand.

### Means for Solving Problem

The present invention is directed to an indwelling needle device, including: a shield that has an inner cavity; a soft outer needle that is fixed to a front end of the shield; a hub that is disposed within the inner cavity of the shield and is movable in a longitudinal direction of the shield; a hard inner needle that is fixed to a front end of the hub; a tube that is connected to a rear end of the hub; and a stopper that can be inserted into or pulled out of the inner cavity of the shield from a rear end of the shield. The hub can be displaced from an initial position at which the inner needle penetrates the outer needle and protrudes from a leading end of the outer needle to a retracted position at which the inner needle is housed within the inner cavity of the shield. When the stopper is inserted into the inner cavity of the shield and a leading end thereof is caused to abut against the hub located at the initial position, a rear end portion of the stopper is exposed behind the shield. The indwelling needle device is configured such that, in a state in which the leading end of the stopper abuts against the hub located at the initial position, the stopper can be pulled out of the shield with one hand, by grasping the rear end portion while pressing a finger against the rear end, or the vicinity thereof, of the shield.

### Effects of the Invention

According to the present invention, not only an insertion operation that inserts an inner needle and an outer needle into a patient but also a housing process that houses the inner needle within a shield can be performed with one hand.

### Brief Description of Drawings

[FIG. 1A] FIG. 1A is a perspective view of an indwelling needle device according to an embodiment of the present invention as seen from above.
[FIG. 1B] FIG. 1B is a perspective view of the indwelling needle device according to the embodiment of the present invention as seen from below.
[FIG. 2] FIG. 2 is a cross-sectional view of the indwelling needle device according to the embodiment of the present invention taken along a vertical plane containing line 2-2 in FIG. 1A and seen in the direction of arrows 2.
[FIG. 3] FIG. 3A is a perspective view of a hub used in the indwelling needle device according to the embodiment of the present invention, FIG. 3B is a cross-sectional view of the hub taken along a plane containing line 3B-3B in FIG. 3A and seen in the direction of arrows 3B, and FIG. 3C is a cross-sectional view of the hub taken along a plane containing line 3C-3C in FIG. 3A and seen in the direction of arrows 3C.
[FIG. 4] FIG. 4A is a perspective view of a stopper used in the indwelling needle device according to the embodiment of the present invention as seen from above, and FIG. 4B is a plan view thereof.
[FIG. 5] FIG. 5 is a perspective view, as seen from above, of the indwelling needle device according to the embodiment of the present invention with the hub being at the retracted position.
[FIG. 6] FIG. 6 is a cross-sectional view of the indwelling needle device according to the embodiment of the present invention taken along a vertical plane containing line 6-6 in FIG. 5 and seen in the direction of arrows 6.
[FIG. 7A] FIG. 7A is a perspective view of a conventional indwelling needle device as seen from above.
[FIG. 7B] FIG. 7B is a perspective view of the conventional indwelling needle device as seen from below.
[FIG. 8] FIG. 8 is a cross-sectional view of the conventional indwelling needle device taken along a vertical plane containing line 8-8 in FIG. 7A and seen in the direction of arrows 8.
[FIG. 9] FIG. 9 is a perspective view of a stopper used in the conventional indwelling needle device shown in FIGS. 7A and 7B.
[FIG. 10] FIG. 10 is a cross-sectional view of the conventional indwelling needle device shown in FIGS. 7A and 7B taken along the same plane as in FIG. 8, with an inner needle being housed within a shield.

### Description of the Invention

The indwelling needle device of the present invention is preferably configured such that an upper face of the rear end portion is formed as an inclined face that is inclined so as to be lower toward the shield. Furthermore, it is preferable that a protrusion that protrudes upward is formed on an outer circumferential face of the shield, at, or in the vicinity of, the rear end thereof. With this configuration, a force easily can be applied by placing a finger on the protrusion formed on the shield.

The indwelling needle device may be configured such that the rear end portion of the stopper includes a pair of grasping portions that sandwich the tube in a horizontal direction. In this case, the pair of grasping portions may be elastically displaceable so as to grip the tube therebetween. With this configuration, the tube disposed between the pair of grasping portions can be gripped via the grasping portions by gripping the pair of grasping portions. Accordingly, the retraction operation can be performed more easily.

Hereinafter, the present invention will be described in detail while showing preferred embodiments thereof. However, it goes without saying that the present invention is not limited to the embodiments below. In the drawings that will be referred to in the following description, only the main members of constituent members of the embodiments of the present invention that are necessary for the description of the present invention are shown in a simplified manner for the sake of convenience of description. Accordingly, the present invention may include optional constituent members that are not shown in the drawings below. Moreover, it should be understood that the dimensions of the members in the drawings below are not a faithful representation of the dimensions of actual constituent members, dimensional ratios of those members, and the like.

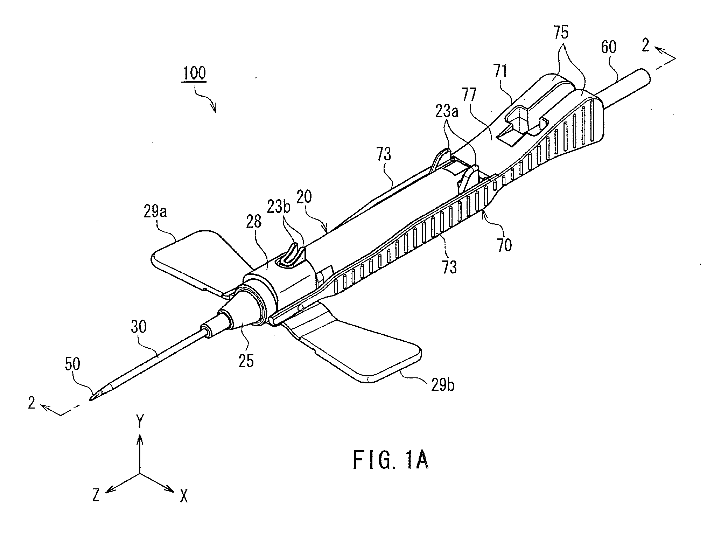

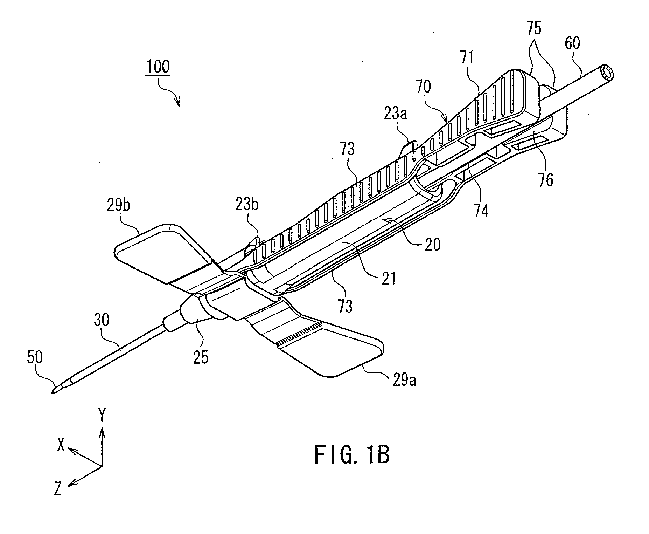

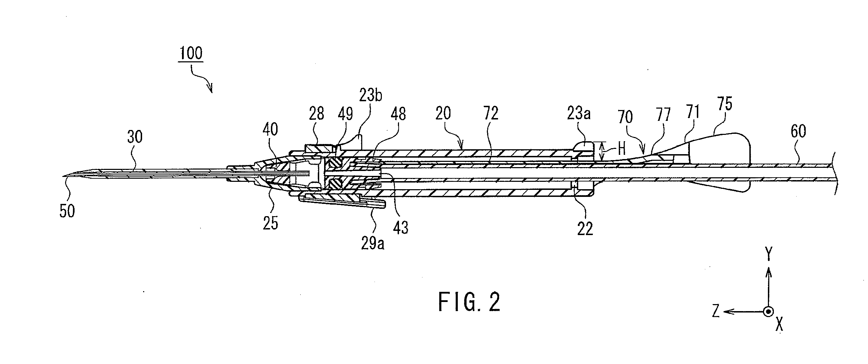

FIG. 1A is a perspective view, as seen from above, of an indwelling needle device 100 according to an embodiment of the present invention with a hub being at the initial position, and FIG. 1B is a perspective view thereof as seen from below. For the sake of convenience of description, an orthogonal coordinate system is set in which the longitudinal direction of the indwelling needle device 100 is taken as a Z axis, and the horizontal axis and the vertical axis orthogonal to the Z axis respectively are taken as an X axis and a Y axis. Furthermore, a side in the direction of the Y axis arrow (i.e., the upper side in FIGS. 1A and 1B) is referred to as an "upper side", and a side that is opposite this side is referred to as a "lower side". Note that the "horizontal direction" and the "vertical direction" do not refer to the actual orientations when using the indwelling needle device 100. Moreover, a side that is inserted into the patient (a side in the direction of the Z axis arrow, that is, the left side in FIGS. 1A and 1B) is referred to as a "front side", and a side that is opposite this side is referred to as a "rear side". FIG. 2 is a cross-sectional view of the indwelling needle device 100 taken along a vertical plane (YZ plane) containing line 2-2 in FIG. 1A and seen in the direction of arrows 2.

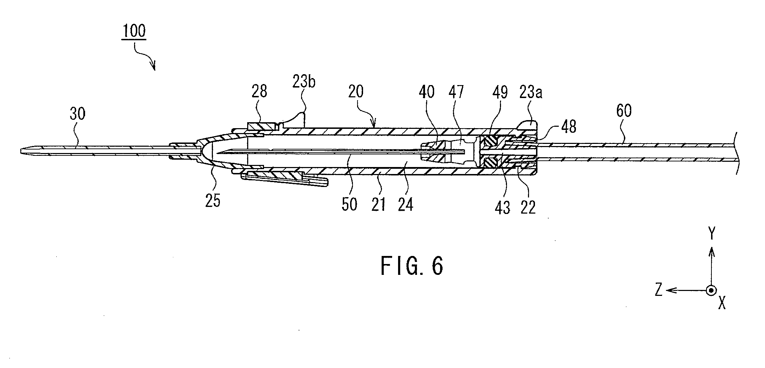

The indwelling needle device 100 includes a shield 20. The shield 20 has a shield tube 21 and an outer hub 25 that is fixed to an end (front end) of the shield tube 21. The shield tube 21 has an approximately cylindrical shape having a constant inner diameter. A pair of protrusions (first protrusions) 23a that protrude upward are formed on the outer circumferential face of the shield tube 21 at an end (rear end) that is opposite the outer hub 25. Furthermore, an engagement protrusion 22 that is continuous in a circumferential direction is formed on an inner circumferential face of the shield tube 21 in the vicinity of an end (rear end) that is opposite the outer hub 25. The outer hub 25 is approximately funnel-shaped, and a soft outer needle 30 is fixed to an end (front end) thereof that is opposite the shield tube 21. The outer needle 30 has an approximately cylindrical shape. Although there is no particular limitation on the materials for the shield tube 21 and the outer hub 25, a hard material is preferable, and, for example, polycarbonate, polypropylene, and the like can be used. Preferably, the shield tube 21 and the outer hub 25 have transparency or translucency, so that fluid (medical fluid, blood, etc.) and a hub 40 inside an inner cavity 24 of the shield 20 (see FIG. 6, which will be described later) can be seen therethrough. Although there is no particular limitation on the material for the outer needle 30, a soft material is preferable, and, for example, polypropylene, polyurethane elastomer, fluororesin such as polytetrafluoroethylene, and the like can be used. Preferably, the outer needle 30 has transparency or translucency, so that fluid (medical fluid, blood, etc.) and an inner needle 50 inside its inner cavity can be seen therethrough. It should be noted that the outer hub 25 and the outer needle 30 also may be integrally formed using the soft material described above.

Reference numerals 29a and 29b indicate wings that extend approximately parallel to the X axis. The wings 29a and 29b are provided on a fixing member 28 having an approximately cylindrical shape. The wings 29a and 29b are installed on the shield 20 by externally fitting the fixing member 28 to the outer circumferential face of the shield tube 21 in the vicinity of its outer hub 25 side end. When a second protrusion 23b that protrudes upward and is formed on the outer circumferential face of the shield tube 21 is fitted to an approximately U-shaped cut-out of the fixing member 28, the fixing member 28 and the wings 29a and 29b are positioned on the shield tube 21. Although there is no particular limitation on the material for the wings 29a and 29b, a soft material is preferable, and, for example, polypropylene, vinyl chloride, polyethylene, olefin or polystyrene thermoplastic elastomer, and the like can be used. It should be noted that the wings 29a and 29b may also be integrally molded with the shield 20.

The hub 40 (inner hub) is inserted in the inner cavity 24 of the shield 20 so as to be movable in a longitudinal direction (i.e., Z axis direction) of the shield 20. The hard inner needle 50 made of metal is fixed to a front end of the hub 40. The inner needle 50 has an approximately cylindrical shape, and the leading end thereof is processed to be sharp. One end of a flexible tube 60 made of resin is connected to a rear end of the hub 40. The other end of the tube 60 is connected to, for example, a drip infusion system for performing infusion. An O-ring 49 is installed on an outer circumferential face of the hub 40. The O-ring 49 is in close contact with the inner circumferential face of the shield tube 21 and prevents, in the inner cavity 24 of the shield 20, medical fluid or blood that is present on the outer needle 30 side with respect to the O-ring 49 from leaking to the tube 60 side with respect to the O-ring 49. Although there is no particular limitation on the material for the hub 40, a hard material is preferable, and, for example, polycarbonate, polypropylene, polyethylene, and the like can be used. Although there is no particular limitation on the material for the tube 60, a soft material is preferable, and, for example, vinyl chloride and the like can be used.

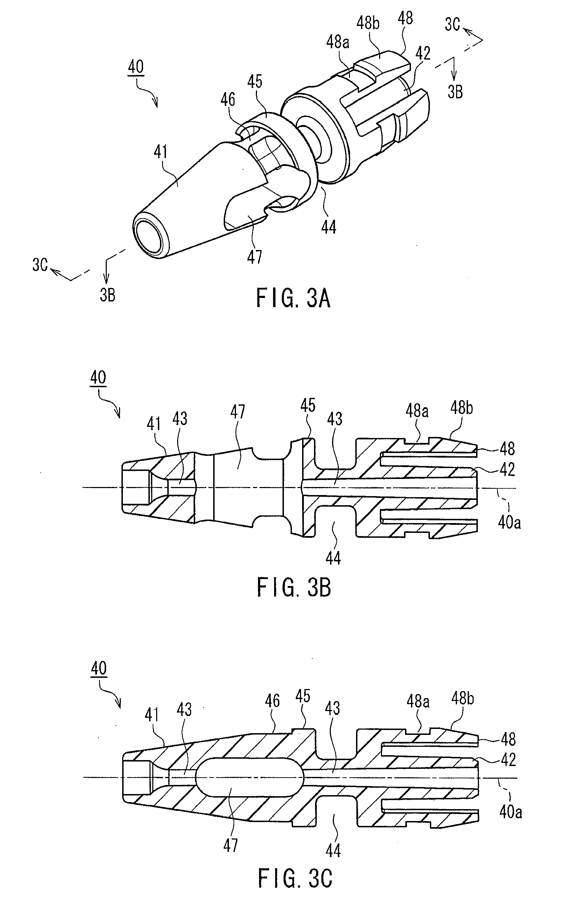

FIG. 3A is a perspective view of the hub 40, FIG. 3B is a cross-sectional view of the hub 40 taken along a plane containing line 3B-3B in FIG. 3A and seen in the direction of arrows 3B, and FIG. 3C is a cross-sectional view of the hub 40 taken along a plane containing line 3C-3C in FIG. 3A and seen in the direction of arrows 3C. The cross-section shown in FIG. 3B and the cross-section shown in FIG. 3C are orthogonal to each other. The hub 40 has at its end (front end) a front portion 41 having a circular conical outer face, and has at its other end a rear portion 42 having a cylindrical outer face. A longitudinal penetration path 43 longitudinally penetrates the hub 40 and extends along a central axis 40a of the hub 40 from the front portion 41 to the rear portion 42. As shown in FIG. 2, the inner needle 50 is inserted into the longitudinal penetration path 43 from the front portion 41 side and held by the hub 40. The rear portion 42 is inserted into the tube 60, so that the hub 40 is connected to the tube 60. Thus, the inner needle 50 and the tube 60 are in communication with each other via the longitudinal penetration path 43 of the hub 40.

An annular groove 44 that is continuous in a circumferential direction is formed in the outer circumferential face of the hub 40 in a location between the front portion 41 and the rear portion 42. As shown in FIG. 2, the O-ring 49 is installed in the annular groove 44.

A large diameter portion 45 and a small diameter portion 46 are formed in that order from the annular groove 44 side, in the outer circumferential face of the hub 40 in respective locations between the annular groove 44 and the front portion 41. The small diameter portion 46 is adjacent to the front portion 41, and the outer diameter of the small diameter portion 46 is substantially the same as the largest diameter of the front portion 41 and is smaller than the outer diameter of the large diameter portion 45. A lateral penetration path 47 that laterally penetrates the front portion 41, the small diameter portion 46, and the large diameter portion 45 in their diameter direction (direction orthogonal to the central axis 40a) is formed in these portions. The lateral penetration path 47 intersects and is in communication with the longitudinal penetration path 43.

Four cantilevered elastic pieces 48 are arranged around the rear portion 42 at equiangular intervals about the central axis 40a of the hub 40. The elastic pieces 48 extend approximately parallel to the central axis 40a of the hub 40. A fitting groove 48a and a tapered surface 48b are formed on a face of each elastic piece 48 that is opposite the rear portion 42. The fitting groove 48a is a recess (groove) extending in the circumferential direction of the hub 40. The tapered surface 48b is adjacent to the fitting groove 48a on a side thereof that is closer to the free end of the elastic piece 48, and constitutes part of a circular conical face having an outer diameter that is larger toward the fitting groove 48a.

In FIGS. 1A, 1B, and 2, the hub 40 is located on the front end side of the inner cavity 24 of the shield 20. In the present invention, this position of the hub 40 relative to the shield 20 is referred to as an "initial position". At the initial position, the inner needle 50 held by the hub 40 penetrates the outer needle 30, and the leading end thereof protrudes to the outside from the leading end of the outer needle 30.

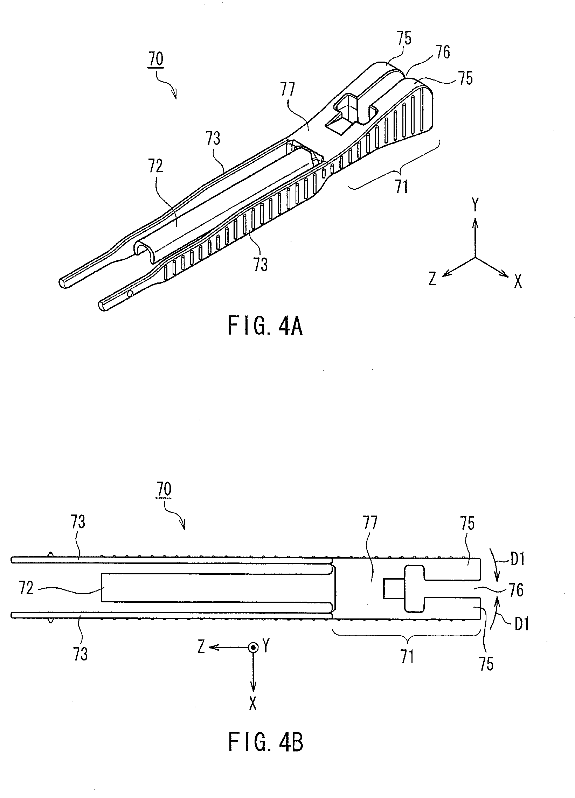

In order to maintain the hub 40 at the initial position, a stopper 70 is used. FIG. 4A is a perspective view of the stopper 70 as seen from above, and FIG. 4B is a plan view thereof. The stopper 70 includes a base portion 71, an insertion portion 72, and a pair of fixing portions 73.

The rear portion of the base portion 71 is divided into a pair of grasping portions 75 along a slit 76 that is formed from the rear end of the base portion 71. The pair of grasping portions 75 face each other in the X axis direction, and can be elastically displaced in mutually approaching directions D1 (see FIG. 4B).

The upper faces of the base portion 71 are formed as inclined faces 77 that are lower toward the insertion portion 72. A height (position in the vertical direction) of the upper faces of the base portion 71 is lowest at the front end of the base portion 71. A dimension in the vertical direction (Y axis direction) of the base portion 71 is larger at the grasping portions 75 than at the portion in front of the grasping portions 75. A groove 74 that is continuous with the slit 76 and that extends in the Z axis direction is formed on the lower face of the base portion 71 (see FIG. 1B).

The insertion portion 72 and the pair of fixing portions 73 extend parallel to the Z axis from the base portion 71 toward the front side. The cross-section of the insertion portion 72 along a plane perpendicular to its longitudinal direction (i.e., plane parallel to the XY plane) is approximately in the shape of a U with an open bottom. The pair of fixing portions 73 are arranged so as to sandwich the insertion portion 72 in the X axis direction. The fixing portions 73 are plate-like members having main faces that are parallel to the YZ plane.

As shown in FIGS. 1A, 1B, and 2, the insertion portion 72 of the stopper 70 is inserted into the inner cavity 24 of the shield 20 from the rear end of the shield tube 21. When the stopper 70 is inserted into the shield 20 as far as possible, the leading end of the insertion portion 72 hits the rear ends of the elastic pieces 48 of the hub 40, the large diameter portion 45 of the hub 40 in turn hits the rear end of the outer hub 25, and the hub 40 is disposed at the initial position within the inner cavity 24 of the shield 20. The tube 60 connected to the hub 40 is fitted to the insertion portion 72 having an approximately U-shaped cross-section, the groove 74 on the lower side of the base portion 71, and the slit 76 between the grasping portions 75. The pair of fixing portions 73 of the stopper 70 are located on both sides of the shield tube 21 of the shield 20, and the leading ends of the fixing portions 73 reach the positions of the wings 29a and 29b. The base portion 71 is exposed behind the shield 20.

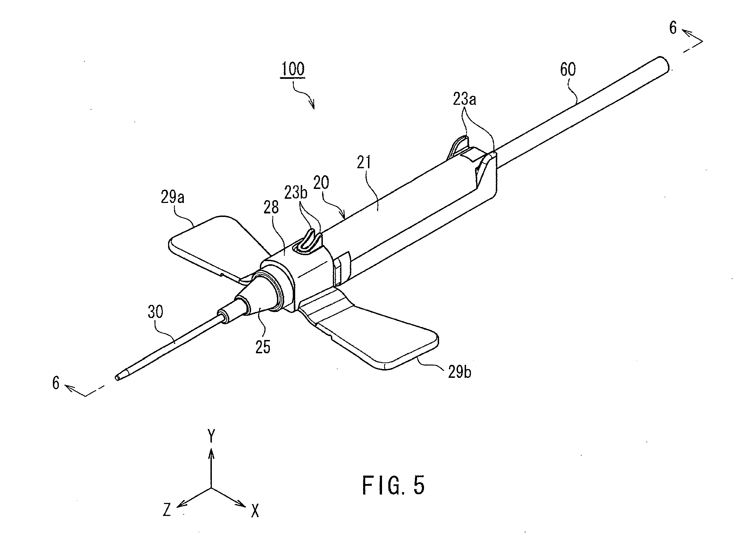

FIG. 5 is a perspective view, as seen from above, of the indwelling needle device 100 with the hub 40 being moved to the retracted position at the rear end inside the inner cavity 24 of the shield 20. FIG. 6 is a cross-sectional view of the indwelling needle device 100 taken along a vertical plane (YZ plane) containing line 6-6 in FIG. 5 and seen in the direction of arrows 6.

As shown in FIG. 6 when the hub 40 is at the retracted position, the fitting grooves 48a (see FIGS. 3A, 3B, and 3C) of the hub 40 and the engagement protrusion 22 of the shield tube 21 are fitted to each other. Moreover, the inner needle 50 held by the hub 40 has been pulled out of the outer needle 30 and is housed within the inner cavity 24 of the shield 20. The stopper 70 has been pulled out of the shield 20 and removed.

When compared with the initial position (see FIGS. 1A, 1B, and 2), at the retracted position, the cross-sectional area of the flow channel within the outer needle 30 is increased by an amount corresponding to the cross-sectional area of the inner needle 50, and accordingly the flow rate of the medical fluid or blood is increased. Furthermore, at the retracted position, the flow channel from the outer needle 30 to the tube 60 includes two flow channels, that is, a first flow channel sequentially passing through the inner cavity of the inner needle 50 and the longitudinal penetration path 43 of the hub 40 and a second flow channel sequentially passing through a space between the inner face of the shield 20 and the respective outer faces of the inner needle 50 and the hub 40, the lateral penetration path 47 of the hub 40, and the longitudinal penetration path 43 of the hub 40, and accordingly the medical fluid or blood can flow at a high flow rate.

As described above, the hub 40 can move from the initial position (FIGS. 1A, 1B, and 2) to the retracted position (FIGS. 5 and 6) inside the inner cavity 24 of the shield 20.

Hereinafter, a method of using the thus configured indwelling needle device 100 of this embodiment will be described.

First, in a state in which the hub 40 is at the initial position and the inner needle 50 protrudes from the leading end of the outer needle 30 as shown in FIGS. 1A, 1B, and 2, the inner needle 50 and the outer needle 30 are inserted into a blood vessel of the patient (insertion operation). At that time, the indwelling needle device 100 is positioned such that its lower face (face opposite the side where the protrusions 23a protrude) faces the patient.

Next, in a state in which the outer needle 30 is inserted in the patient, the inner needle 50 is retracted (retraction operation). That is to say, the tube 60 is pulled out of the shield 20, so that the hub 40 connected to the front end of the tube 60 and the inner needle 50 held by the hub 40 are moved toward the rear side relative to the shield 20. As the hub 40 moves, the stopper 70 moves toward the rear side.

The engagement protrusion 22 is formed on the inner circumferential face of the shield tube 21 in the vicinity of its rear end. The hub 40 moves to the engagement protrusion 22, and the tapered surfaces 48b formed on the respective outer faces of the elastic pieces 48 of the hub 40 slide on the engagement protrusion 22. At this time, the elastic pieces 48 undergo elastic deformation to the rear portion 42 side. Then, when the tapered surfaces 48b have passed over the engagement protrusion 22, the elastic pieces 48 undergo elastic recovery, and the engagement protrusion 22 is fitted to the fitting grooves 48a. In this manner, the hub 40 moves to the retracted position shown in FIGS. 5 and 6.

In this state, an adhesive tape is attached to the skin of the patient over the wings 29a and 29b, and the indwelling needle device 100 is fixed to the patient. Only the outer needle 30 is left inside the patient in a state in which it is inserted in the patient. At the retracted position, the hard inner needle 50 is not present in the flexible outer needle 30, and therefore, even if the position of the indwelling needle device 100 relative to the patient changes due to movement of the patient or the like, the outer needle 30 does not injure the blood vessel and the like of the patient.

When the necessary treatment has been finished, the adhesive tape that fixes the wings 29a and 29b is removed from the patient, and the outer needle 30 is withdrawn from the patient. Even when the tube 60 is pushed or pulled relative to the shield 20, the fitted state in which the fitting grooves 48a of the hub 40 and the engagement protrusion 22 of the shield tube 21 are fitted to each other is not released. That is to say, the inner needle 50 cannot protrude again from the leading end of the outer needle 30, and the inner needle 50 cannot be withdrawn from the shield 20 together with the hub 40. Accordingly, accidental puncture with the hard inner needle 50 and accidental reuse of the used indwelling needle device 10 are prevented. The used indwelling needle device 100 will be discarded.

In the above-described insertion operation, when the inner needle 50 and the outer needle 30 are inserted into the patient, the inner needle 50 receives a reaction force. Thus, the inner needle 50 and the hub 40 holding the inner needle 50 have to be prevented from being moved toward the rear side by this reaction force relative to the outer needle 30 and the shield 20. The leading end of the insertion portion 72 of the stopper 70 abuts against the rear end (the elastic pieces 48) of the hub 40, and restricts the movement of the hub 40. It is necessary that, during puncture, the operator grips the indwelling needle device 100 such that the stopper 70 does not move relative to the shield 20.

For example, the pair of grasping portions 75 of the stopper 70 can be gripped with two fingers in the horizontal direction (X axis direction). Specifically, the indwelling needle device 100 can be held by gripping the pair of grasping portions 75 with the thumb and the middle finger and placing the index finger on the upper face (e.g., the protrusions 23a or the second protrusion 23b) of the shield 20. This holding method is preferable because the indwelling needle device 100 can be held stably. Since the pair of grasping portions 75 are elastically displaced in mutually approaching directions (see arrows D1 in FIG. 4B) by the horizontal gripping force applied to the pair of grasping portions 75, the pair of grasping portions 75 grasp the tube 60 located therebetween. Thus, according to the holding method of the indwelling needle device 100 described above, the tube 60 can be gripped via the pair of grasping portions 75.

In the following retraction operation, in a state in which the pair of grasping portions 75 are still gripped with the thumb and the middle finger in the horizontal direction, the index finger is pressed against the protrusions 23a of the shield 20. Then, the stopper 70 is pulled out of the shield 20 toward the rear side while the protrusions 23a is pushed with the index finger such that the shield 20 does not move relative to the patient. Since the tube 60 is grasped by the pair of grasping portions 75, the tube 60 is pulled out of the shield 20 together with the stopper 70. When the stopper 70 is substantially completely pulled out of the shield 20, the hub 40 has been moved to the retracted position (see FIGS. 5 and 6).

In this manner, according to the indwelling needle device 100 of this embodiment, the protrusions 23a that protrude upward are formed at the rear end of the shield 20. Accordingly, a force easily can be applied to the shield 20 by placing the index finger on the protrusions 23a. Thus, in the retraction operation, the hub 40 easily can be moved to the retracted position, by pushing the protrusions 23a with the index finger while gripping the pair of grasping portions 75 with the thumb and the middle finger in the horizontal direction. Accordingly, the retraction operation can be performed with one hand.

If the insertion operation is performed while gripping the pair of grasping portions 75 with the thumb and the middle finger as described above, the retraction operation can be performed consecutively without switching the indwelling needle device 100 between fingers. Accordingly, the insertion operation and the retraction operation can be performed continuously only with one hand.

Moreover, if the insertion operation is performed while placing the index finger on the protrusions 23a, the indwelling needle device 100 can be held stably, and, furthermore, the retraction operation can be performed without changing the position of the index finger.

The upper faces of the base portion 71 are formed as the inclined faces 77 that are inclined so as to be gradually lower toward the shield 20. As shown in FIGS. 2 and 4A, the inclined faces 77 extend to the front end of the base portion 71, and the front end of the inclined faces 77 (or the base portion 71) has a height substantially the same as that of the upper face of the insertion portion 72. Accordingly, as shown in FIG. 2, an upward protrusion height H of the protrusions 23a relative to the front end of the inclined faces 77 can be made large. Thus, when the index finger is placed on the inclined faces 77 and slid toward the front side, the index finger quite naturally hits the protrusions 23a. Since the protrusion height H of the protrusions 23a is large, a force easily can be applied by pressing a finger against the protrusions 23a. This configuration is advantageous for improving the efficiency of the retraction operation and for stably holding the indwelling needle device 100 in the insertion operation.

Furthermore, since the inclined faces 77 are formed on the base portion 71, the dimension in the vertical direction of the pair of grasping portions 75 can be made large while suppressing the height of the front end of the base portion 71 to a low height substantially similar to that of the upper face of the insertion portion 72. This configuration is advantageous for stably holding the indwelling needle device 100 in the insertion operation and the retraction operation because, when the pair of grasping portions 75 are gripped in the horizontal direction, the area of regions in contact with the fingers increases.

The foregoing embodiment should be considered as illustrative only. The present invention is not limited to the foregoing embodiment, and can be modified as appropriate.

The configuration of the stopper is not limited to that described in the foregoing embodiment.

For example, although the slit 76 is formed in the base portion 71 in the foregoing embodiment, the slit 76 may be omitted. In this case, it is difficult to grasp the tube 60 together with the stopper 70 even by gripping the base portion 71 in the horizontal direction. However, in the retraction operation, it is possible to pull the stopper 70 and the tube 60 together out of the shield 20 as in the foregoing embodiment, by directly holding the tube 60 between the little finger and/or the ring finger and the palm of the hand holding the base portion 71. Accordingly, the retraction operation can be performed with one hand. Holding the tube 60 between the little finger and/or the ring finger and the palm is effective also in the foregoing embodiment in which the slit 76 is formed. The reason for this is that directly holding the tube 60 in this manner can prevent the tube 60 from slipping relative to the pair of grasping portions 75, so that the hub 40 can be reliably moved to the retracted position.

Although the slit 76 extends parallel to the Z axis in the foregoing embodiment, the shape of the slit is not limited thereto. For example, the slit may be inclined with respect to the Z axis, or may have a bent or curved portion. If the tube 60 is fitted to such a slit, slippage of the tube 60 relative to the pair of grasping portions 75 in the retraction operation can be reduced.

The inclined faces do not have to be formed throughout the upper faces of the base portion 71. For example, the upper faces of the grasping portions 75 may be on a plane approximately parallel to the XZ plane, and the inclined faces may be formed only in a region in front of these upper faces. It is preferable that, among the upper face of the portion (rear end portion), exposed behind the shield 20, of the stopper 70, the portion that is closest to the shield 20 is lowest.

The inclined faces do not have to have flat surfaces, and may have curved surfaces, or have flat surfaces and curved surfaces in any combination.

The pair of fixing portions 73 may be omitted. The rear end portion of the stopper 70 exposed behind the shield 20 when the insertion portion 72 is inserted in the shield 20 does not have to have a width (dimension in the X axis direction) larger than that of the insertion portion 72, as in the base portion 71 described in the foregoing embodiment, and, for example, may have a width substantially the same as that of the insertion portion 72.

Although the protrusions 23a are formed at the rear end of the shield 20 in the foregoing embodiment, the protrusions 23a do not have to be formed at the rear end of the shield 20, and may be formed at a position in front of the rear end. However, if the protrusions 23a are formed at a front side position too away from the rear end of the shield 20, the index finger does not reach the protrusions 23a in the retraction operation, and, thus, the efficiency of the retraction operation deteriorates. Accordingly, it is preferable that the protrusions 23a are formed at, or in the vicinity of, the rear end of the shield 20. That is, the distance from the rear end of the shield 20 to the protrusions 23a may be not greater than 20 mm, more specifically not greater than 10 mm, and particularly specifically not greater than 5 mm. Furthermore, the distance from the rear end of the shield 20 to the protrusions 23a may be specifically not greater than 1/2 the length of the shield 20, more specifically not greater than 1/3 the length, and particularly specifically not greater than 1/4 the length.

Although two protrusions 23a are formed facing each other in the X axis direction in the foregoing embodiment, the number of protrusions 23a may be one, or three or more protrusions may be arranged side by side in the X axis direction. There is no limitation on the shape or dimension of the protrusions 23a. The area of a region in contact with the index finger may be increased such that a force can be easily applied by the index finger, and a region in contact with the index finger may be provided with a rough surface so that slippage of the index finger can be prevented. The shape of regions, other than the region in contact with the index finger, of the protrusions may be freely changed. For example, the front portions of the protrusions may extend to the front side with a gentle slope or at substantially the constant height.

The structure for fitting the hub 40 located at the retracted position and the shield 20 to each other also may have a configuration other than the above-described configuration. Alternatively, the fitting structure may be omitted.

Although the method for holding the indwelling needle device 100 in the insertion operation was described in the foregoing embodiment in which the pair of grasping portions 75 are gripped with the thumb and the middle finger in the horizontal direction and the index finger is placed on the upper face of the shield 20, the method for holding the indwelling needle device 100 in the insertion operation is not limited thereto. For example, the indwelling needle device 100 can be held using any conventional methods. Some holding methods in the insertion operation require the indwelling needle device 100 to be switched between fingers when the operation shifts from the insertion operation to the retraction operation, but, even in these cases, the insertion operation and the retraction operation can be performed with the same one hand.

### Industrial Applicability

There is no particular limitation on the field of use of the present invention, and the present invention can be extensively used as an indwelling needle device for use in such treatments as infusion, blood transfusion, extracorporeal blood circulation, and the like. Among these, the present invention preferably can be used as an indwelling needle device for infusion or hemodialysis.

### List of Reference Numerals

- 20: Shield
- 21: Shield tube
- 23a: Protrusion
- 24: Inner cavity of shield
- 25: Outer hub
- 30: Outer needle
- 40: Hub
- 50: Inner needle
- 60: Tube
- 70: Stopper
- 71: Base portion (rear end portion)
- 72: Insertion portion
- 73: Fixing portion
- 74: Groove
- 75: Grasping portion
- 76: Slit
- 77: Inclined face
- 100: Indwelling needle device

## Claims

1. An indwelling needle device, comprising:
a shield that has an inner cavity;
a soft outer needle that is fixed to a front end of the shield;
a hub that is disposed within the inner cavity of the shield and is movable in a longitudinal direction of the shield;
a hard inner needle that is fixed to a front end of the hub;
a tube that is connected to a rear end of the hub; and
a stopper that can be inserted into or pulled out of the inner cavity of the shield from a rear end of the shield;
wherein the hub can be displaced from an initial position at which the inner needle penetrates the outer needle and protrudes from a leading end of the outer needle to a retracted position at which the inner needle is housed within the inner cavity of the shield,
when the stopper is inserted into the inner cavity of the shield and a leading end thereof is caused to abut against the hub located at the initial position, a rear end portion of the stopper is exposed behind the shield, and
in a state in which the leading end of the stopper abuts against the hub located at the initial position, the stopper can be pulled out of the shield with one hand, by grasping the rear end portion while pressing a finger against the rear end, or the vicinity thereof, of the shield.

2. The indwelling needle device according to claim 1,
wherein an upper face of the rear end portion is formed as an inclined face that is inclined so as to be lower toward the shield, and
a protrusion that protrudes upward is formed on an outer circumferential face of the shield, at, or in the vicinity of, the rear end thereof.

3. The indwelling needle device according to claim 1 or 2,
wherein the rear end portion of the stopper includes a pair of grasping portions that sandwich the tube in a horizontal direction, and
the pair of grasping portions can be elastically displaced so as to grip the tube therebetween.
